# EUROPÄISCHE PATENTSCHRIFT

(11) **EP 3 061 489 B1**
(45) Veröffentlichungstag und Bekanntmachung des Hinweises auf die Patenterteilung: **11.09.2019**
(21) Anmeldenummer: 16155908.3
(22) Anmeldetag: 16.02.2016
(51) Int. Cl.: A61M 39/10, A61M 1/14, A61M 39/20, A61M 39/24, A61M 39/26

(54) **KONNEKTOR FÜR EINEN DIALYSATOR**
CONNECTOR FOR A DIALYSIS MACHINE
CONNECTEUR POUR UN DIALYSEUR

(30) Priorität: 25.02.2015 DE 102015102719
(43) Veröffentlichungstag der Anmeldung: 31.08.2016
(73) Patentinhaber: B. Braun Avitum AG, 34212 Melsungen (DE)
(72) Erfinder: GEIGER, Ralph, 34587 Felsberg (DE); SCHLITT, Christof, 34621 Frielendorf (DE)
(74) Vertreter: Winter, Brandl, Fürniss, Hübner, Röss, Kaiser, Polte - Partnerschaft mbB

(56) Entgegenhaltungen:
- WO-A1-2006/122406
- WO-A1-2009/063281
- DE-A1- 4 203 417
- DE-A1-102014 100 326
- DE-C1- 3 513 205
- US-A1- 2004 068 238
- US-A1- 2011 306 940

## Beschreibung

Die Erfindung betrifft eine Konnektoreinheit zum Verbinden eines Anschlussports eines Dialysators mit einer flüssigkeitsführenden Leitung, wobei die Konnektoreinheit einen durchgehenden Strömungskanal aufweist und einerseits des Strömungskanals einen Kupplungsabschnitt zur flüssigkeitsdichten Verbindung mit dem Anschlussport des Dialysators und andererseits des Strömungskanals einen Kupplungsabschnitt zur flüssigkeitsdichten Verbindung mit der flüssigkeitsführenden Leitung aufweist. Sie betrifft des Weiteren ein Verfahren zum Spülen eines Filterelements, insbesondere eines Dialysators für extrakorporale Blutbehandlungsverfahren, wie bspw. für Dialyse, Hämofiltration oder Ultrafiltration, unter Verwendung einer solchen Konnektoreinheit.

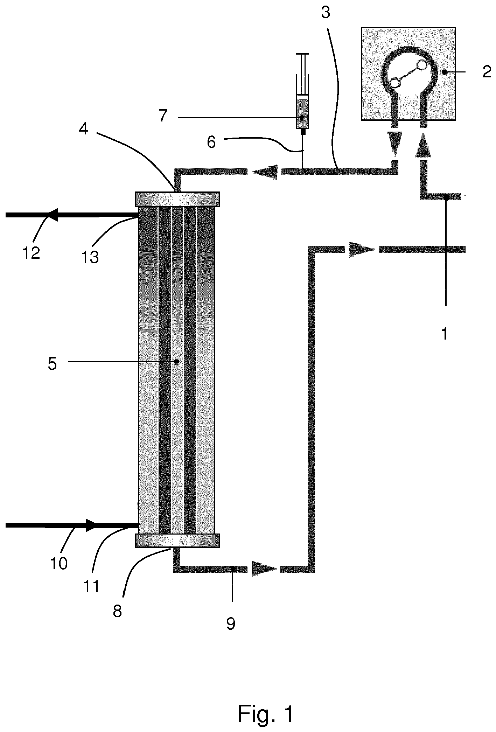

Bekannte Dialysatoren weisen in der Regel vier Anschlüsse auf, mit denen sie einerseits mit einem Leitungssystem einer Dialysemaschine und anderseits mit einem mit einem Patienten verbundenen extrakorporalen Blutsystem angeschlossen werden. Die genannten Anschlüsse teilen sich auf in zwei Anschlüsse zur Verbindung mit dem extrakorporalen Blutsystem, über die zu reinigendes Blut zum Dialysator hin und vom Dialysator abgeführt wird, im Folgenden auch als Blutanschlüsse bezeichnet, und zwei Anschlüsse zur Verbindung mit der Dialysemaschine, über die Dialysierflüssigkeit zum Dialysator hin und vom Dialysator abgeführt wird, im Folgenden auch als Dialysierflüssigkeitsanschlüsse bezeichnet (siehe schematische Darstellung der Fig. 1).

Vor dem Beginn eines Blutbehandlungsverfahrens ist es notwendig, den extrakorporalen Blutkreislauf einschließlich des Dialysatorfilters zu füllen und zu spülen, um die im extrakorporalen Kreislauf vorhandene Luft und ggf. Rückstände im Dialysator zu entfernen. Das Spülen geschieht in der Regel mit einer Kochsalzlösung. Hierzu wird ein vorgefüllter Beutel mit Kochsalzlösung verwendet, der manuell an das arterielle Blutschlauchende angeschlossen wird. Die Blutpumpe der Dialysemaschine fördert dann die Flüssigkeit durch den extrakorporalen Kreislauf. Die Kochsalzlösung tritt schließlich am venösen Ende des Blutschlauches in einen Abfallbeutel aus. Nach ausreichend umgewälzter Flüssigkeit ist der extrakorporale Kreislauf gespült und befüllt und damit für die Blutbehandlung vorbereitet.

Um ein Eindringen von Verunreinigungen in den Dialysator und ein Auslaufen von darin ggf. vorhandener Flüssigkeit zu vermeiden sowie die Anschlüsse vor Beschädigung zu schützen, werden die Anschlüsse des Dialysators zunächst abgedeckt und verschlossen.

Für den oben beschriebenen Spülvorgang müssen die Kappen, welche die Blutanschlüsse des Dialysators verschließen, entfernt werden. Diese werden in der Regel dann an den Dialysierflüssigkeitsanschlüssen angebracht. Nach dem Spülen werden die Kappen von den Dialysierflüssigkeitsanschlüssen entfernt, um daran die Zu- bzw. Ableitungen des Dialysierflüssigkeitsschlauchsystems anbringen zu können.

Es gibt Lösungen, bei denen alle vier Anschlüsse eines Dialysators separat mit jeweils einer Kappe verschlossen sind. Zum Beispiel ist aus der DE 38 255 73 A1 eine Verschlusskappe für Dialysatoren zur Verwendung bei deren Sterilisation, mit einem ersten und einem zweiten Kappenteil bekannt, von denen das erste Kappenteil an einem Dialysatorflansch anbringbar ist. Das zweite Kappenteil ist zwischen einer Offenstellung, in der der Durchtritt eines Sterilisationsmediums erfolgt, und einer Schließstellung bewegbar. Das erste Kappenteil weist eine Außenhülse, die den Dialysatorflansch im Einbauzustand steril dichtend außen übergreift, und ein rückwärtiges Teil auf, das mit einer In-line-Sterilisationseinrichtung in Strömungsverbindung bringbar ist.

Aus der EP 1 000 632 A2 ist des Weiteren bekannt ein Verschlusselement zum sterilen Verschließen von Anschlüssen von Filtermodulen für Dialyse, Hämofiltration oder Ultrafiltration (eine sogenannte Sterilbarriere), welches Verschlusselement eine Wandung umfasst, die einen selbsttätig schließenden schlitzförmigen Einschnitt aufweist, der im geschlossenen Zustand keimdicht abschließt. Es sind Befestigungsmittel vorgesehen, die an die Wandung angrenzen und mittels derer das Verschlusselement mit einem Anschluss verbindbar ist.

Aus der US 2009/0004053 A1 ist ein System bekannt, mit welchem eine medizinische Einrichtung automatisch gereinigt, desinfiziert, getestet und aktiviert werden kann. Das System umfasst einen Dialysator mit Bluteinlass und Blutauslass, eine Blutauslassleitung zur Verbindung mit dem Blutauslass mit einem maschinenseitigen und einem patientenseitigen Ende, eine Bluteinlassleitung zur Verbindung mit dem Bluteinlass mit einem maschinenseitigen und einem patientenseitigen Ende, ein Anschlussstück zur Verbindung mit dem Dialysator und eine Wiederverwendungseinrichtung zum Paaren mit dem Dialysator. Das Anschlussstück umfasst mehrere Verbinder, die zur Verbindung mit den patientenseitigen Enden der Blutein- und der Blutauslassleitung ausgebildet sind. Die Wiederverwendungseinrichtung ist zur Paarung mit dem Anschlussstück ausgebildet und eingerichtet, die Bluteinlassleitung, die Blutauslassleitung und den Dialysator zu reinigen.

Aus der WO 2009/063281 A1 ist ein medizinischer Verbinder zum Verbinden von einer Dialysatzuleitung oder einer Dialysatableitung mit einem Fluidanschluss einer Dialysemaschine bekannt. Der Verbinder besitzt eine sichtbare Signaleinrichtung, die aus einem farbigen Streifen gebildet ist, der auf einem Zapfen angeordnet ist. Das Signal ist sichtbar, wenn sich der Zapfen in einer korrekten Eingriffsstellung mit einem externen Element befindet. Andernfalls ist der das Signal tragende Abschnitt des Zapfens abgesenkt, so dass das Signal nicht sichtbar ist und eine unkorrekte Verbindung anzeigt. Der Fluidanschluss der Dialysemaschine ist mit einem Sperrventil versehen.

Aus der WO 2005/046785 A1 ist ein Konnektor zur Verbindung eines Dialysatports eines Blutdialysators mit einer Dialysat führenden Leitung bekannt. Der Konnektor weist einen ihn durchlaufender Hohlraum, ein den Hohlraum umschließendes erstes Ende, das geeignet ist, in den Hohlraum den Dialysatport aufzunehmen, und ein den Hohlraum umschließenden zweites Ende, das geeignet ist, mit der Dialysat führenden Leitung verbunden zu werden, auf. Am ersten Ende ist eine Aussparung mit einem von der Aussparung aufgenommenen Schiebeelement vorgesehen. Das Schiebeelement ist zwischen einer ersten und einer zweiten Position senkrecht zur Richtung des Hohlraums im ersten Ende verschiebbar. Das Schiebeelement durchdringt in der ersten Position den Hohlraum des ersten Endes nicht und engt in der zweiten Position den Hohlraum des ersten Endes ein, so dass der Konnektor mit dem Schiebeelement in der ersten Position auf den Dialysatport aufgesteckt wird und in der zweiten Position durch eine Hinterschneidung am Dialysatport an diesem arretiert werden kann.

DE 42 03 417 A1 2 offenbart eine Schnellkupplung mit einem Durchlass, der mittels eines federvorgespannten Ventils abgesperrt ist, wenn kein Stecker mit der Schnellkupplung gekuppelt ist. Bei eingeschobenen Stecker wird das Ventil gegen die Spannung der Feder durch Kontakt zwischen dem Stecker und dem Schließkörper, des Ventils geöffnet.

US 2004/0068238 A1 offenbart einen Injektionszugang mit einem üblichen Lüer-Lock-Connector. Der Durchflussquerschnitt des Injektionszugangs ist mit einer Elastomerscheibe verschlossen. Diese ist mit einem Schlitz versehen. Ohne Fluidverbindung ist der Schlitz verschlossen. Nach Erstellen der Fluidverbindung ist die Scheibe durch den Connector zwar vorgespannt, dichtet aber immer noch ab, solang eingangsseitig kein Fluiddruck anliegt. Ausströmen von Fluid ist nicht möglich. Liegt nach Erstellen der Fluidverbindung eingangsseitig Fluiddruck an, öffnet sich der Schlitz und Fluid kann einströmen.

US 2001/0306940 A1 zeigt einen männlichen Kupplungsabschnitt, der mit einem weiblichen Kupplungsabschnitt fluiddicht verbunden wird und ein Ventil aufweist. Das Ventil liegt bei nicht vorhandener Fluidverbindung in geschlossenem Zustand vor und wird beim Anschluss der Kupplungsabschnitte und geöffnet, indem die Nadel beim Anschließen von einem Septum zurückgedrückt wird und die Oberfläche mit dem Ventil so zusammenwirkt, dass die beiden Ventilflügel einen Kanal freigeben.

WO 2006/122406 A1 zeigt eine Kupplung mit einem männlichen Teil und einem weiblichen Teil, die fluiddicht zusammengeschlossen werden, wobei das männliche Teil ein Ventil aufweist, das unterhalb eines vorbestimmten Drucks die Leitung absperrt.

DE 35 13 205 C1 offenbart einen Konnektor für eine Peritonealdialyse, der eine Kupplung mit einem weiblichen Abschnitt und einem männlichen Abschnitt mit einer Ventilplatte, die bei nicht vorhandener Fluidverbindung den Strömungskanal absperrt, aufweist.

DE 10 2014 100 326 A1 zeigt eine Fluidkupplung mit einen männlichen Verbindungsabschnitt, einem weiblichen Verbindungsabschnitt und einem Ventilkörper, der durch eine Vorspannfeder mit dem männlichen Abschnitt verbunden ist und durch Auflage auf dem Ventilsitz bei nicht vorhandener Fluidverbindung den Strömungskanal absperrt. Bei Verbinden des Leitungsabschnitts mit dem männlichen Verbindungsabschnitt wird der Stab des Ventilkörpers vom Ventilsitz weggeschoben und gibt einen Strömungskanal frei.

Die aus dem Stand der Technik bekannte Verwendung von Kappen zum Verschließen von Dialysierflüssigkeitsanschlüssen eines Dialysators hat mehrere Nachteile:
Bei dem vorstehend beschriebenen Wechseln der Kappen zum Spülen des Dialysators kann es in nachteiliger Weise zu Verschmutzung, Verformungen oder dem Verlust der Kappen kommen. Des Weiteren kann der Fall eintreten, dass die Kappen nicht wieder korrekt geschlossen werden. Wenn dem Anwender beim Wechsel eine Kappe herunterfällt, ist diese verschmutzt und kann nicht mehr verwendet werden. Wird sie dennoch irrtümlich verwendet, kann die Kappe den Anschluss und somit die Dialysierflüssigkeit verschmutzen, der Filter ist somit nicht mehr nutzbar. Eine Verwendung des Dialysefilters ist in einem solchen Fall nicht mehr möglich.

Schließlich muss nach einem Spülen des Dialysators mit Spüllösung beim Anschließen von Dialysierflüssigkeitsleitungen an die Dialysierflüssigkeitsanschlüsse des Dialysators die Kappe entfernt werden. Hierbei tritt in der Regel noch im Dialysator vorliegende Spüllösung aus. Es wurde beobachtet, dass Anwender, um einen solchen Austritt zu minimieren oder zu verhindern, teilweise die Dialysierflüssigkeitsanschlüsse beispielsweise mit einem Finger temporär verschließen. Dies führt in nachteiliger Weise zu einer weiteren Möglichkeit von Kontamination.

Ein weiterer Nachteil ist, dass der Zeitpunkt des Entfernens der Kappen unterschiedlich ist und von Anwender zu Anwender unterschiedlich gehandhabt wird. Dadurch können die Anschlüsse (sowohl Blutanschlüsse als auch Dialysierflüssigkeitsanschlüsse) offen, d.h. ungeschützt an der Maschine vorhanden sein.

Ausgehend von dem vorstehend beschriebenen Stand der Technik liegt der vorliegenden Erfindung die Aufgabe zugrunde, die zuvor angeführten Nachteile zu beseitigen, insbesondere ein System zur Verfügung zu stellen, bei dem ein Entweichen von Flüssigkeit bei nicht mit dem Dialysator verbundenen Flüssigkeitsleitungen, zum Beispiel beim Anschließen des Dialysators, insbesondere von Spülflüssigkeit nach einem Spülen des Dialysators, verhindert werden kann. Vorzugsweise soll das System auf bestehende medizintechnische Einrichtungen adaptierbar sein, also auch bei bekannten Dialysatoren und Konnektionssystemen ein Entweichen von Flüssigkeit bei entkoppeltem Dialysator verhindern. Des Weiteren soll sichergestellt sein, dass auch bei entkoppeltem Dialysator dieser steril verschlossen ist.

Die Aufgabe wird durch die Merkmale des Anspruchs 1 gelöst. Vorteilhafte Weiterbildungen sind Gegenstand von Unteransprüchen.

Erfindungsgemäß wird ein Konnektor zum Verbinden eines Anschlusses eines Dialysators mit einer flüssigkeitsführenden Leitung bereitgestellt. Bei der flüssigkeitsführenden Leitung kann es sich um eine Blutzuleitung, eine Blutableitung, eine Dialysierflüssigkeitszuleitung und eine Dialysierflüssigkeitsableitung handeln. Der Konnektor weist einen Kupplungsabschnitt, insbesondere einen männlichen Hansenkonnektorabschnitt, zur flüssigkeitsdichten Verbindung mit einem Anschlusselement, insbesondere einer weiblichen Hansenkonnektoraufnahme, der entsprechenden Leitung auf. In einem Strömungskanal des Konnektors ist ein Absperrorgan oder Flusswiderstand integriert, das bzw. der unterhalb eines vorbestimmten Drucks und/oder bei nicht vorliegender (Fluid-)Verbindung zwischen dem Anschluss und der Leitung, d.h. wenn die Leitung nicht mit dem Konnektor verbunden ist, den Strömungsquerschnitt des Strömungskanals fluiddicht absperrt und das Absperrorgan strömungsrichtungsunabhängig zu öffnen ist, und das bei vorliegender Verbindung zwischen dem Anschluss und der Leitung, d.h. wenn die Leitung mit dem Konnektor verbunden ist oder wird und/oder ein Fluidstrom mit einem vorbestimmten Druck oder höher aufgebaut wird, den Strömungsquerschnitt des Strömungskanals freigibt, d.h. einen Fluidstrom zwischen Dialysatoranschluss und Leitung zulässt.

Dies bedeutet, dass der Konnektor den Strömungskanal verschließt, wenn keine Leitung angeschlossen ist bzw. noch keine Leitung angeschlossen worden ist. Der Strömungskanal ist zunächst verschlossen. Somit muss der Dialysator nicht mit einem zusätzlichen Verschluss oder Kappe verschlossen werden. Somit entfällt auch eine mögliche Gefahr einer Kontamination über die Kappe. Ferner wird dadurch verhindert, dass zwischen dem Entfernen der Kappe und dem Anschließen der Spüllösungsleitung der Anschluss, wenn auch kurz, offen und somit ungeschützt ist. Somit kann der Dialysator ohne Kappen, aber dennoch fluiddicht ausgeliefert bzw. vertrieben werden. Nach dem Spülen kann sichergestellt werden, dass die im Dialysator befindliche Spüllösung vor dem Anschließen der Dialysierflüssigkeitsab- und -zuleitungen nicht ausläuft. Durch die strömungsrichtungsunabhängige Öffnung kann das Absperrorgan unabhängig von der jeweiligen Anschlusskonfiguration und der dadurch bedingten Strömungsrichtung geöffnet werden.

An den Konnektoren der Dialysierflüssigkeitszuleitung und Dialysierflüssigkeitsableitung, welche beim Spülvorgang offen bleiben, kann der Grenzdruck, ab welchen das jeweilige Absperrorgan öffnet, entsprechend so voreingestellt bzw. einstellbar sein, dass dieser höher als der beim Spülvorgang auf der Dialysierflüssigkeitsseite des Dialysators zu erwartende Fluiddruck ist.

Der Konnektor kann in Form eines Adapters oder einer Konnektoreinheit, d.h. eines separaten Bauteils, ausgebildet sein, das an dem Anschluss(port) des Dialysators anbringbar ist, oder Teil des Anschlussports des Dialysators selbst sein bzw. mit diesem einstückig ausgebildet sein.

Durch die Erfindung wird ein Konnektionssystem zum Anschließen von flüssigkeitsführenden Leitungen an entsprechende Ports eines Dialysators geschaffen, wobei das Konnektionssystem selbst Mittel enthält, die ein Ausströmen von Flüssigkeit verhindern, wenn keine dichte strömungstechnische Verbindung zwischen dem Dialysator und der flüssigkeitsführenden Leitung vorliegt. Im Falle einer separaten Konnektoreinheit ist diese je nach Ausbildung ihrer beiderseitigen Kupplungsabschnitte mit beliebigen Dialysatoren und Flüssigkeitsleitungen verwendbar, unabhängig von der Ausgestaltung der daran jeweils vorliegenden Anschlüsse.

Erfindungsgemäß ist die Konnektoreinheit an einen Anschlussport eines Dialysators gekuppelt bzw. wird durch einen Anwender daran gekuppelt. Sobald die Konnektoreinheit und der Dialysator miteinander gekuppelt sind, besteht zwischen dem entsprechenden Anschluss des Dialysators und dem Strömungskanal der Konnektoreinheit eine strömungstechnische Verbindung. Diese ist mittels des im Strömungskanal erfindungsgemäß vorliegenden Sperrorgans gesperrt, solange nicht eine Flüssigkeitsleitung mit der Konnektoreinheit auf der dem Dialysator gegenüberliegenden Seite gekuppelt, insbesondere flüssigkeitsdicht gekuppelt ist. Der Strömungskanal in der Konnektoreinheit ist durch die Konnektoreinheit durchgehend ausgebildet und führt anders ausgedrückt von einer Konnektoreingangsseite zu einer Konnektorausgangsseite. Je nach Durchströmungsrichtung der Konnektoreinheit kann die Konnektoreingangsseite oder die Konnektorausgangsseite eingerichtet sein, um mit dem Anschlussport des Dialysators flüssigkeitsdicht verbunden werden zu können, und die Konnektorausgangsseite bzw. die Konnektoreingangsseite eingerichtet sein, um mit der flüssigkeitsführenden Leitung flüssigkeitsdicht verbunden werden zu können.

Der Flusswiderstand bzw. das Absperrorgan ist derart ausgebildet, dass der Strömungskanal in dem Konnektor bzw. der Konnektoreinheit entsperrt ist bzw. wird, sobald eine Leitung an dem Dialysator angeschlossen ist und Flüssigkeit durch den Dialysator gepumpt wird. Damit bietet die Erfindung ein System, mit dem jeder Dialysator besonders einfach und effektiv abgedichtet sein bzw. werden kann, solange dieser nicht mit einer Leitung verbunden ist, welche Abdichtung zudem besonders einfach durch Erstellen einer strömungstechnischen Verbindung zwischen dem Dialysator und der flüssigkeitsführenden Leitung entsperrt werden kann. Dies ist in vorteilhafter Weise unabhängig vom jeweiligen Dialysator. Das erfindungsgemäße System lässt sich nämlich sowohl mit solchen Dialysatoren nutzen und verwenden, bei denen alle Anschlüsse oder ein Teil der Anschlüsse bereits mit einem diese selektiv sperrenden Portsystem versehen sind, als auch mit solchen Dialysatoren, bei denen kein Anschluss mit einer derartigen selektiven Abdichtung versehen ist. Dadurch ist die erfindungsgemäße Konnektoreinheit hochflexibel durch einen Anwender des Dialysators einsetzbar.

Zum Beispiel wird zumindest ein Blutanschluss oder werden beide Blutanschlüsse eines Dialysators mit jeweils einer Konnektoreinheit nach der Erfindung gekuppelt bzw. deren Anschlüsse entsprechend ausgestaltet. Vorzugsweise wird zusätzlich oder alternativ ein Dialysierflüssigkeitsanschluss oder werden beide Dialysierflüssigkeitsanschlüsse des Dialysators ebenfalls mit jeweils einer Konnektoreinheit nach der Erfindung gekuppelt. Bei einem in der Regel vor einer Behandlung durchzuführenden Spülvorgang oder Primingvorgang können nun Flüssigkeitsleitungen in beliebiger Weise mit dem leitungsseitigen Kupplungsabschnitt der jeweiligen Konnektoreinheit gekuppelt und entkuppelt werden, ohne dass bei entkuppelter Leitung Flüssigkeit aus dem Dialysator ausströmen kann. Die aus dem Stand der Technik bekannte und eingangs beschriebene Verwendung von Kappen zum Verschluss der Dialysatoranschlüsse ist damit überflüssig.

Durch die Erfindung kann insgesamt eine deutliche Erhöhung der Sicherheit einer extrakorporalen Blutbehandlung gegen eine Infektion des Patienten durch Kontamination bewirkt werden. Des Weiteren kann ein vereinfachtes Handling für den Anwender, verbunden mit einer geringeren Möglichkeit von Kontamination bewirkt werden. Schließlich können Fehlbedienungen vermieden werden. Letztendlich können Kosteneinsparungen bewirkt werden, da auf die aus dem Stand der Technik bekannten und dort erforderlichen Kappen verzichtet werden kann. Ein besonderer Vorteil aber ist, dass das erfindungsgemäße Konnektorsystem ja nach Ausbildung eine zusätzliche Sterilbarriere darstellen kann. Es kann insbesondere ein zusätzlicher Sauerstoffausschluss aus dem Dialysatorfilter erzielt werden.

Vorteilhafte Ausführungsformen der Erfindung sind unter anderem in den Unteransprüchen beansprucht und werden nachfolgend näher erläutert.

Unter einem Dialysator im Sinne der vorliegenden Beschreibung der Erfindung ist insbesondere ein Filtermodul für die extrakorporale Blutbehandlung zu verstehen. Unter einer flüssigkeitsführenden Leitung im Sinne der vorliegenden Beschreibung der Erfindung ist insbesondere eine Fluidzuleitung oder Fluidableitung zu verstehen, insbesondere eine Fluidzuleitung oder Fluidableitung einer Vorrichtung zur extrakorporalen Blutbehandlung, wie zum Beispiel eine Dialysemaschine.

Von den Kupplungsabschnitten der Konnektoreinheit kann zumindest einer als Schnellverbindungskupplungsabschnitt, zum Beispiel als Hansenkupplungselement, ausgebildet sein. Vorzugsweise weist ein Kupplungsabschnitt der Konnektoreinheit eine weibliche Konnektoraufnahme auf, insbesondere eine weibliche Hansenkonnektoraufnahme. Diese ist zur Verbindung mit einem männlichen Gegenelement, insbesondere mit einem männlichen Hansenkonnektorelement ausgebildet und geeignet. Insbesondere der anschlussportseitige Kupplungsabschnitt des Konnektors kann mit einer solchen weiblichen Konnektoraufnahme, insbesondere einer weiblichen Hansenkonnektoraufnahme ausgebildet sein, so dass die Konnektoreinheit mit einem oftmals als männliches Gegenelement, insbesondere als männliches Hansenkonnektorgegenelement, ausgebildeten Anschlussport des Dialysators nach Art einer Hansen-Kupplung zu verbinden ist. Eine solche Verbindung kann in vorteilhafter Weise besonders einfach durch einen Anwender erstellt und gelöst werden und ist im Bereich von extrakorporalen Blutbehandlungseinrichtungen weit verbreitet, so dass von einer hohen Kompatibilität der Konnektoreinheit nach der Erfindung ausgegangen werden kann.

Nach einer Ausführungsform der Erfindung weist die Konnektoreinheit zwei miteinander kuppelbare sowie voneinander entkuppelbare Konnektorelemente auf. Dabei kann insbesondere das eine Konnektorelement zur gegenseitigen Kopplung mit dem anderen Konnektorelement einen männlichen Kupplungsabschnitt und das andere Konnektorelement zur gegenseitigen Kopplung mit dem einen Konnektorelement einen weiblichen Kupplungsabschnitt aufweisen. Auf diese Weise sind die beiden Konnektorelemente der Konnektoreinheit besonders einfach und anwenderfreundlich miteinander kuppelbar und voneinander entkuppelbar. In zumindest einem der Konnektorelemente, vorzugsweise in dem dialysatorseitigen Konnektorelement, ist ein Absperrorgan nach der Erfindung angeordnet. Eine solche Ausbildung der Konnektoreinheit birgt besondere Vorteile in einem Fall, wenn ein Dialysator verwendet wird, dessen Anschlussports keine Sterilbarrieren oder ähnliche die Anschlüsse des Dialysators sperrende Einheiten aufweist. Die Konnektoreinheit wird in der zuvor beschriebenen Weise an einem Anschluss des Dialysators angekoppelt. Im Falle eines Spülvorgangs oder einer Primingprozedur verbleibt das anschlussportseitige Konnektorelement mit dem Anschlussport des Dialysators gekoppelt, während die Flüssigkeitsleitung von diesem getrennt wird, indem die beiden Konnektorelemente voneinander entkoppelt werden. Im Ergebnis kann auch bei einem nicht mit Sperreinrichtungen versehenen Dialysator ein Umstecken von Flüssigkeitsleitungen, zum Beispiel von den Blutanschlüssen auf die Dialysierflüssigkeitsanschlüsse und umgekehrt, erfolgen, ohne dass Flüssigkeit aus dem Dialysator austreten kann oder dessen Anschlüsse bei gelösten Flüssigkeitsleitungen offen und kontaminierbar wären. Man kann auch sagen, dass eines der Konnektorelemente als eine Art Adapter wirkt, der durch einen Anwender lösbar mit dem Konnektor verbindbar ist.

Die entsprechenden Kupplungsabschnitte der Konnektoreinheit bzw. der Konnektorelemente können insbesondere als männliches Hansenkupplungselement und weibliches Hansenkupplungselement ausgebildet sein. Nach einer Ausführungsform besitzt jedes der beiden Konnektorelemente einen weiblichen Kupplungsabschnitt sowie einen männlichen Kupplungsabschnitt.

Nach einer Ausführungsform der Erfindung ist das Absperrorgan druckabhängig zu öffnen. Im Falle von entkoppelten Flüssigkeitsleitungen ist die an dem Absperrorgan anliegende Druckdifferenz derart klein oder nicht vorhanden, dass das Absperrorgan den Strömungskanal absperrt und keine Flüssigkeit durch- und damit ausströmen kann. Bei angekuppelter Flüssigkeitsleitung liegt an dem Absperrorgan eine zu dessen Öffnen ausreichende Druckdifferenz an, und zwar unabhängig davon, ob es sich bei dem jeweiligen Anschluss um einen Zufluss oder Abfluss handelt. Diese Lösung ist sehr anwenderfreundlich.

Nach einer weiteren Ausführungsform der Erfindung weist das Absperrorgan ein Rückschlagventil auf. Auf diese Weise kann die Konnektoreinheit bzw. der Konnektor gezielt mit nur einer Durchflussrichtung ausgebildet werden, was Fehlbedienungen zu vermeiden hilft. Um beide Flussrichtungen zu ermöglichen, kann das Absperrorgan auch als bidirektionales Rückschlagventil ausgebildet sein.

Das Absperrorgan ist vorzugsweise bei allen Ausführungsformen derart beschaffen, dass es mehrfach verwendbar ist, d.h. bei gekoppelter Flüssigkeitsleitung offen ist und bei entkoppelter Flüssigkeitsleitung sperrt, dies vorzugsweise weitgehend unabhängig von der Anzahl bereits getätigter Kopplungsvorgänge.

Bei einer Ausführungsform kann die Konnektoreinheit ein Öffnungselement zum Öffnen des Absperrorgans aufweisen. Das Öffnungselement ist vorzugsweise in einem der beiden Konnektorelemente angeordnet. Es ist derart ausgebildet, dass es beim Koppeln der Flüssigkeitsleitung an den Dialysator das Absperrorgan zwangsläufig durchsticht und öffnet. Das Öffnungselement ist vorzugsweise als Anstechelement ausgebildet. Vorzugsweise ist das weibliche Konnektorelement ausgebildet, das Absperrorgan zu aktivieren.

Anders ausgedrückt betrifft die Erfindung die Integration eines Absperrorgans in ein Konnektorsystem, zum Beispiel durch einen Konnektor mit zumindest einer der folgenden Eigenschaften:
- Der Konnektor macht eine Verwendung von Kappen sowie ein Zuhalten und Abdichten von diskonnektierten Anschlüssen per Daumen obsolet.
- Der Konnektor kann aus Rückschlagventilen bestehen und mit allen Geräteherstellern kompatibel sein.
- Der Konnektor kann von weiblichem Hansen-Konnektor aktiviert werden. Die Erfindung schließt einen Konnektor an einem männlichem Konnektor und einen Aktivator an einem weiblichem Hansen-Konnektor ein (weiblicher Hansen-Konnektor bleibt kompatibel zu anderen männlichen Konnektoren).
- Der Konnektor kann druckabhängig und richtungsunabhängig öffnen.

Bei den vorgenannten Ausführungsformen kann der Konnektor immer mit dem Dialysator statisch verbunden sein und weist ein Absperrorgan auf. Der Anwender muss keine zusätzlichen manuellen Handhabungsschritte durchführen.

Die Erfindung betrifft außerdem ein Verfahren zum Spülen eines Dialysators für eine extrakorporale Blutbehandlung, welcher Dialysator einen Blutzuleitungsanschluss, einen Blutableitungsanschluss, einen Dialysierflüssigkeitszuleitungsanschluss und einen Dialysierflüssigkeitsableitungsanschluss aufweist, wobei an zumindest einem Anschluss des Dialysators, vorzugsweise an jedem Anschluss des Dialysators, ein Konnektor nach einem der vorstehenden Ansprüche, angeordnet ist und der Dialysator gespült wird, indem eine Spülflüssigkeitszuleitung mit dem an den Blutzuleitungsanschluss angeordneten Konnektor gekoppelt wird und eine Spülflüssigkeitsableitung mit dem an den Blutableitungsanschluss angeordneten Konnektor gekoppelt wird.

Durch das Verfahren nach der Erfindung ist sichergestellt, dass bei einem Entkoppeln oder Lösen einer der Flüssigkeitsleitungen stets die erfindungsgemäße Konnektoreinheit oder zumindest das den Absperrorgan beinhaltende Konnektorelement an dem entsprechenden Anschluss des Dialysators gekoppelt verbleibt und so ein Auslaufen von Flüssigkeit daraus verhindert. Anders ausgedrückt wird durch ein statisch mit dem Dialysator verbundenes Absperrorgan ein Austreten von Flüssigkeit, insbesondere von Spüllösung beim initialen Spülen, verhindert. Der Anwender muss hierzu keine weiteren Handhabungsschritte aktiv durchführen. Dies führt zu erhöhter Sicherheit, vereinfachter Anwendbarkeit und geringeren Verunreinigungen.

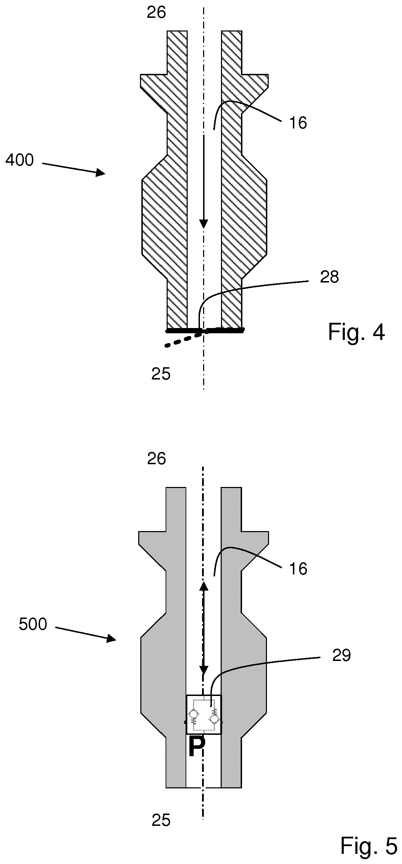

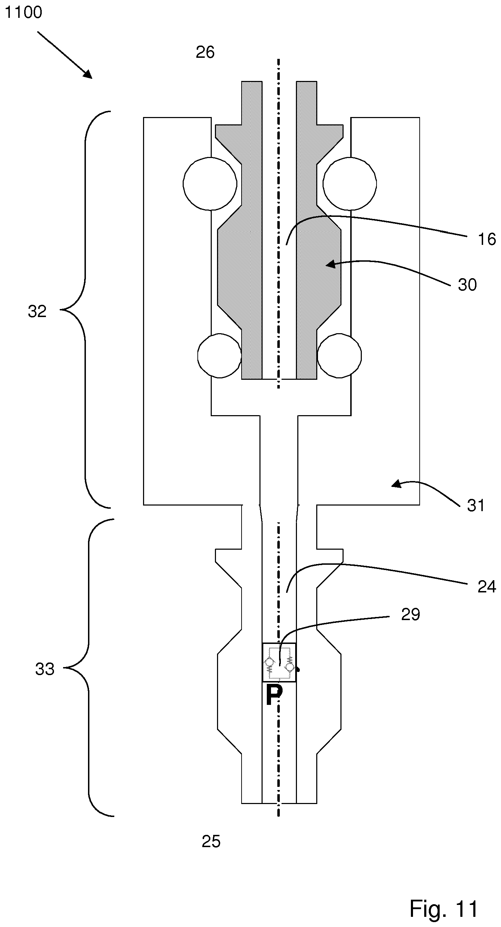

Die Erfindung wird im Folgenden anhand beispielhafter, nicht einschränkender und in den angehängten Figuren 5 und 11 gezeigter Ausführungsformen näher erläutert. Es zeigt:
Figur 1 eine schematische Darstellung eines Fluidsystems einer Vorrichtung zur extrakorporalen Blutbehandlung,
Figur 2 eine schematische Darstellung einer Dialysator-Verbindung nach dem Stand der Technik,
Figur 3 eine schematische Darstellung einer nicht beanspruchten Ausführungsform in einer Schnittansicht,
Figur 4 eine schematische Darstellung einer nicht beanspruchten Ausführungsform in einer Schnittansicht,
Figur 5 eine schematische Darstellung einer Ausführungsform der Erfindung in einer Schnittansicht,
Figur 6 eine schematische Darstellung einer nicht beanspruchten Ausführungsform in einer Schnittansicht,
Figur 7 eine schematische Darstellung einer nicht beanspruchten Ausführungsform in einer Schnittansicht,
Figur 8 eine schematische Darstellung einer nicht beanspruchten Ausführungsform in einer Schnittansicht,
Figur 9 eine schematische Darstellung einer nicht beanspruchten Ausführungsform in einer Schnittansicht,
Figur 10 eine schematische Darstellung einer nicht beanspruchten Ausführungsform in einer Schnittansicht,
Figur11 eine schematische Darstellung einer weiteren Ausführungsform der Erfindung in einer Schnittansicht und
Figur 12 eine schematische Darstellung einer nicht beanspruchten Ausführungsform in einer Schnittansicht.

Figur 1 zeigt beispielhaft einen Ausschnitt einer Vorrichtung zur extrakorporalen Blutbehandlung, hier einer Dialysevorrichtung. Gezeigt ist im Wesentlichen der gesamte extrakorporale Blutkreislauf der Vorrichtung. Dieser weist eine arterielle Blutleitung 1 auf, mittels der Blut von einem nicht gezeigten Patienten zu einer Peristaltikpumpe 2 der Behandlungsvorrichtung geführt wird. Hochdruckseitig der Peristaltikpumpe 2 führt eine Hochdruckblutleitung oder Blutzuleitung 3 unter Hochdruck stehendes aber noch unbehandeltes Blut zu einem Blutzuleitungsanschluss 4 eines Dialysators 5. Hinter der Peristaltikpumpe 2 können mittels einer Zuleitung 6 und einer Pumpe 7 Zusatzstoffe in das im System befindliche Blut gegeben werden, z.B. Gerinnungshemmer oder Heparin zur Blutverdünnung. Im Dialysator 5 wird Blut in bekannter Weise mittels der Dialysierflüssigkeit behandelt, z.B. gereinigt. Behandeltes Blut wird über einen Blutableitungsanschluss 8 vom Dialysator 5 über eine venöse Blutleitung oder Blutableitung 9 zurück zum Patienten geleitet.

Dem Dialysator 5 wird frische Dialysierflüssigkeit über eine Dialysierflüssigkeitszuleitung 10, die mit einem Dialysierflüssigkeitszuleitungsanschluss 11 des Dialysators 5 gekoppelt ist, zugeführt. Verbrauchte Dialysierflüssigkeit wird über eine Dialysierflüssigkeitsableitung 12, die mit einem Dialysierflüssigkeitsableitungsanschluss 13 des Dialysators 5 gekoppelt ist, aus diesem entfernt und einer nicht dargestellten Entsorgung oder Aufbereitung zugeführt. Aus Figur 1 geht hervor, dass der Dialysator 5 im Gegenstrom von Blut und Dialysierflüssigkeit durchströmt wird.

Vor Beginn einer Behandlung wird der Dialysators 5 mit einer Spüllösung über die Blutanschlüsse 4 und 8 gespült. Hierzu wird in der Regel ein vorgefüllter Beutel mit Kochsalzlösung manuell an das arterielle Blutschlauchende angeschlossen und mittels der Peristaltikpumpe 2 diese durch den extrakorporalen Kreislauf einschließlich des Dialysators 5 gepumpt. Die Kochsalzlösung tritt schließlich am venösen Ende des Blutschlauches 9, z. B in einen Abfallbeutel aus. Nach ausreichend umgewälzter Flüssigkeit ist der extrakorporale Kreislauf gespült und befüllt und damit für die Blutbehandlung vorbereitet.

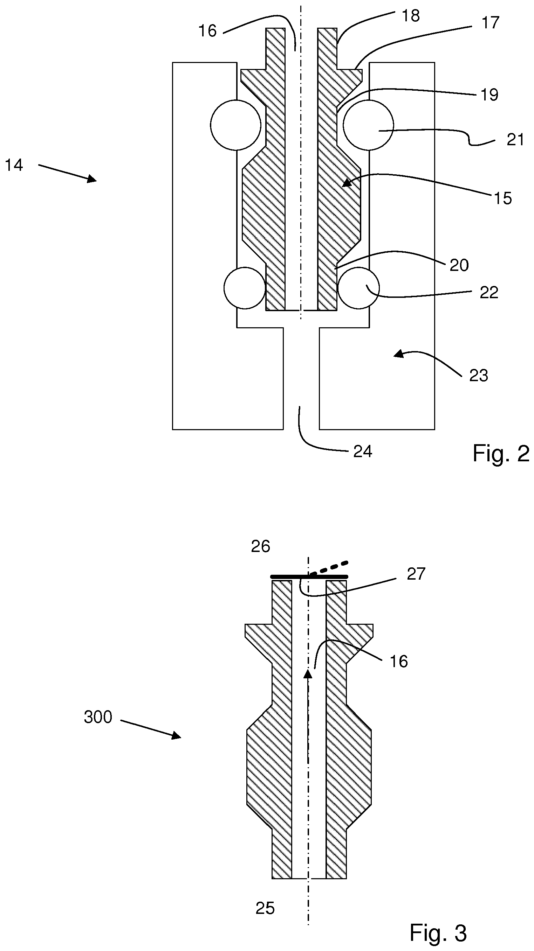

Figur 2 zeigt beispielhaft und schematisch einen Konnektor 14 nach dem Stand der Technik. Dieser weist ein einen Anschluss oder Anschlussport des Dialysators 5 ausbildendes Anschlusselement 15 in Form eines männlichen Hansenkonnektorelements auf. Dieses ist ein an sich bekanntes hohlzylinderartiges Element mit einem durchgehenden Strömungskanal 16. An der dialysatornahen Seite ist das Anschlusselement 15 mit einem von einer Schulter 17 begrenzten Zapfen 18 versehen, der einer dauerhaften Anordnung des Anschlusselements 15 am Dialysator 5 dient. Auf der vom Zapfen 18 abgewandten Seite der Schulter 17 ist eine umlaufende Ringnut 19 in die Außenkontur des Anschlusselements 15 eingebracht. Leitungsseitig ist ein stufenförmiger endseitiger Absatz 20 in die Außenkontur des Anschlusselements 15 eingebracht. Die Nut 19 und Absatz 20 dienen der Aufnahme von Kopplungselementen in Form von Kugeln 21, 22, die in radialer Richtung beweglich radial nach innen vorgespannt, jedoch in axialer Richtung fixiert in einem weiblichen Hansenkonnektorelement 23 aufgenommen sind. In diesem ist ein durchgehender Strömungskanal 24 ausgebildet. Das weibliche Hansenkonnektorelement 23 ist auf der dem männlichen Anschlusselement 15 gegenüberliegenden Seite fest mit einer der Flüssigkeitsleitungen 3, 9, 10, 12 verbunden. Aus Figur 2 geht offensichtlich hervor, dass bei einer Diskonnektion der Flüssigkeitsleitung 3, 9, 10, 12 vom Dialysator durch Entkoppeln des weiblichen Hansenkonnektorelements 23 vom Anschlusselement 15 dessen Strömungskanal 16 offen ist und bleibt, so dass in nachteiliger Weise Flüssigkeit aus dem Inneren des Dialysators 5 herausfließen kann und des Weiteren Verschmutzungen in das Innere des Dialysators 5 eindringen und diesen kontaminieren können.

Die Figuren 3 bis 12 zeigen verschiedene Ausführungsformen von Konnektoren, wobei nur die Ausführungsformen der Figuren 5 und 11 beansprucht werden und wobei in den dargestellten Beispielen als Kupplungsprinzip stets das eines Hansenkonnektors genutzt wird. Dieses wurde vorstehend bereits mit Bezug zum Stand der Technik beschrieben und ist allgemein bekannt, so dass auf eine weitere diesbezügliche Beschreibung bei den nachfolgenden Beispielen verzichtet wird und auf die vorstehenden Ausführungen verwiesen wird. Es sei angemerkt, dass die Erfindung auch mit anderen Kupplungssystemen und Verbindungen genutzt werden kann und nicht auf die Verwendung eines Hansenkonnektors beschränkt ist. Ferner kann an Stelle eines männlichen Hansenkonnektorelements ein weibliches Hansenkonnektorelement vorgesehen sein und umgekehrt.

Die Figur 3 zeigt einen Konnektor 300 in Form eines männlichen Hansenkonnektors 300, der ähnlich wie in der Fig. 2 Teil eines nicht dargestellten eingangsseitigen Anschlusses oder Anschlussports 4, 11 des Dialysators 5, über welchen dem Dialysator Blut, Spüllösung oder Dialysierflüssigkeit zugeführt wird. Die Strömungsrichtung ist in der Figur 3 entsprechend mit einem Pfeil gekennzeichnet. Auf der dem Dialysator 5 abgewandten Seite 25 (unten in Figur 3) ist ein weibliches Anschlussstück 23 auf den Konnektor 300 von der Leitungsseite 25 her aufschiebbar und koppelbar. Der Strömungskanal 16 des Konnektors 300 ist auf der dem Dialysator 5 zugewandten Ende 26 des Konnektors 300 mit einem Absperrorgan in Form eines Rückschlagventils 27 verschlossen, das auf der stromabwärtigen Seite 25 bzw. Dialysatorseite des Konnektors 300 angeordnet oder ausgebildet ist. Das Rückschlagventil 27 sperrt in Ausströmrichtung, d.h. aus dem Dialysator 5 heraus, und gibt den Strömungskanal 16 in Einströmrichtung, d.h. in den Dialysator 5 hinein, frei. Das Rückschlagventil 27 sperrt fluiddicht ab, solange eine an ihm anliegenden Druckdifferenz kleiner als ein bestimmter Grenzwert ist. Bei Überschreiten des Grenzwerts öffnet das Rückschlagventil 27 und gibt den Strömungskanal 16 frei. Das Rückschlagventil 27 ist so eingestellt bzw. vorgespannt, dass es erst beim Betrieb der Pumpe 2 und durch den zu erwartenden Pumpendruck öffnet.

Figur 4 zeigt einen dem Konnektor 300 der Figur 3 ähnelnden Konnektor 400 in Form eines männlichen Hansenkonnektors 400, der ähnlich wie in der Fig. 2 Teil eines nicht dargestellten ausgangsseitigen Anschlusses oder Anschlussports 8, 13 des Dialysators 5, über welchen Blut bzw. Spüllösung oder Dialysierflüssigkeit vom Dialysator 5 abgeführt wird. Die Strömungsrichtung ist in der Figur 4 entsprechend mit einem Pfeil gekennzeichnet und ist entgegengesetzt zur Strömungsrichtung in der Figur 3. Auf der dem Dialysator 5 abgewandten Seite (unten in Figur 3) ist ein weibliches Anschlussstück 8, 13 auf den Konnektor 400 von der Flüssigkeitsleitungsseite 25 her aufschiebbar und koppelbar. Der Strömungskanal 16 des Konnektors 400 ist mit einem Absperrorgan in Form eines Rückschlagventils 28 verschlossen, das auf der stromabwärtigen Seite bzw. Flüssigkeitsleitungsseite 25 des Konnektors 400 angeordnet oder ausgebildet ist. Das Rückschlagventil 28 sperrt in Einströmrichtung, d.h. in den Dialysator 5 hinein, und gibt den Strömungskanal 16 in Ausströmrichtung, d.h. aus dem Dialysator 5 heraus, frei, wobei das Rückschlagventil 28 nicht von alleine nach außen öffnet, sondern nur bei entsprechendem Förderdruck, so dass es ohne Pumpbetrieb den Anschlussport 8 bzw. 13 fluiddicht verschließt.

Figur 5 zeigt einen Konnektor 500, der im Wesentlichen eine Kombination der beiden in den Figuren 3 und 4 gezeigten Ausführungsformen darstellt. Anstelle eines bei entsprechendem Druck nach innen öffnenden Rückschlagventils 27 oder eines bei entsprechendem Druck nach außen öffnenden Rückschlagventils 28 ist in dem Strömungskanal 16 ein bidirektionales Rückschlagventil bzw. Ventilsystem 29 vorgesehen, das druckabhängig und richtungsunabhängig arbeitet. Das Ventilsystem 29 sperrt sowohl in Ausströmrichtung als auch in Einströmrichtung, solange eine an ihm anliegenden Druckdifferenz kleiner als ein bestimmter Grenzwert ist. Bei Überschreiten des Grenzwerts öffnet das Ventilsystem 29 und gibt den Strömungskanal 16 in Richtung der Druckdifferenz, d.h. von der Hochdruckseite in Richtung der Niederdruckseite, frei. Mit anderen Worten der Konnektor 500 eignet sich, unabhängig davon, ob er sich an einem Zulauf oder Ablauf des Dialysators 5 befindet. Ein weibliches Anschlussstück 4, 8, 11, 13 ist wiederum auf der Flüssigkeitsleitungsseite 25 aufschiebbar und koppelbar.

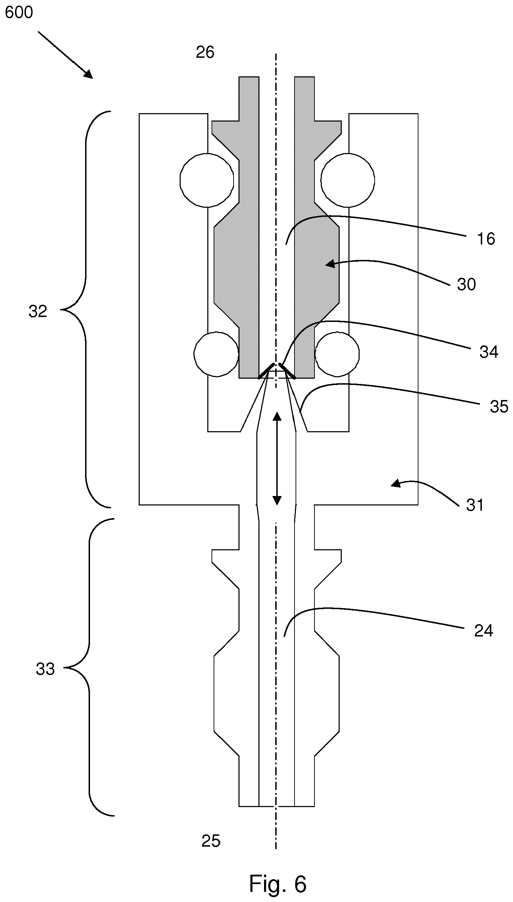

Die Figur 6 zeigt einen Konnektor 600. Dieser weist ein männliches Konnektorelement 30 in Form eines männlichen Hansenkonnektors, der ähnlich wie in der Fig. 2 Teil eines nicht dargestellten eingangs- oder ausgangsseitigen Anschlusses oder Anschlussports 4, 8, 11, 13 des Dialysators 5 ist, und ein Konnektorelement 31 mit einem weiblichen Kupplungsabschnitt 32 und einem männlichen Kupplungsabschnitt 33, beide jeweils nach Art eines Hansenkonnektors, auf. Der männliche Kupplungsabschnitt 33 des Konnektorelements 31 ist mit einem in Figur 5 nicht dargestellten weiblichen Hansenkonnektorelements 23 einer der Leitungen 3, 9, 10, 12 koppelbar. Die Strömungsrichtung ist in der Figur 6 wieder gekennzeichnet, wobei der Konnektor 600 sowohl an einem eingangseitigen Anschluss 4, 11 als auch an einem ausgangsseitigen Anschluss 8, 13 mit entsprechend umgekehrter Strömungsrichtung vorgesehen sein kann bzw. sich hierfür eignet. In dem Konnektorelement 30 ist ein Strömungskanal 16 und in dem Konnektorelement 31 ist ein Strömungskanal 24 ausgebildet.

An seiner Eingangsseite 25 (Leitungsseite 25) ist das Konnektorelement 30 mit einem Absperrorgan in Form eines, insbesondere mechanisch betätig- oder verschiebbaren, Schließelements 34 verschlossen. Das Schließelement ist auf der dem Konnektorelement 31 zugewandten Seite des Konnektorelements 30 angeordnet oder ausgebildet. Das Schließelement 34 ist selbsttätig geschlossen und sperrt damit den Strömungskanal 16 bei Diskonnektion in beide Richtungen fluiddicht ab. Er gibt den Strömungskanal 16 in beide Richtungen frei, sobald eine Konnektion durch bestimmungsgemäßes Koppeln von Konnektorelement 30 und Konnektorelement 31 bewirkt ist. Dies wird durch ein Öffnungselement 36, das am Konnektorelement 31 ausgebildet ist, bewirkt, indem dieses bei bestimmungsgemäßer Konnektion das Schließelement 34 in eine geöffnete Stellung drängt oder betätigt. Bei dem Öffnungselement handelt es sich um einen dem Konnektorelement 30 zugewandten Vorsprung, der in den Strömungskanal 16 des Konnektorelements 30 eindringt und dort das darin vorgesehene Schließelement 34 oder ein ähnliches Sperrorgan betätigt oder aktiviert, so dass es den Strömungskanal 16 für beide Strömungsrichtungen freigibt. Ein weibliches Anschlussstück 4, 8, 11, 13 des Dialysators 5 ist auf den Kupplungsabschnitt 33 des Konnektorelements 31 von der Flüssigkeitsleitungsseite 25 aufschiebbar und koppelbar.

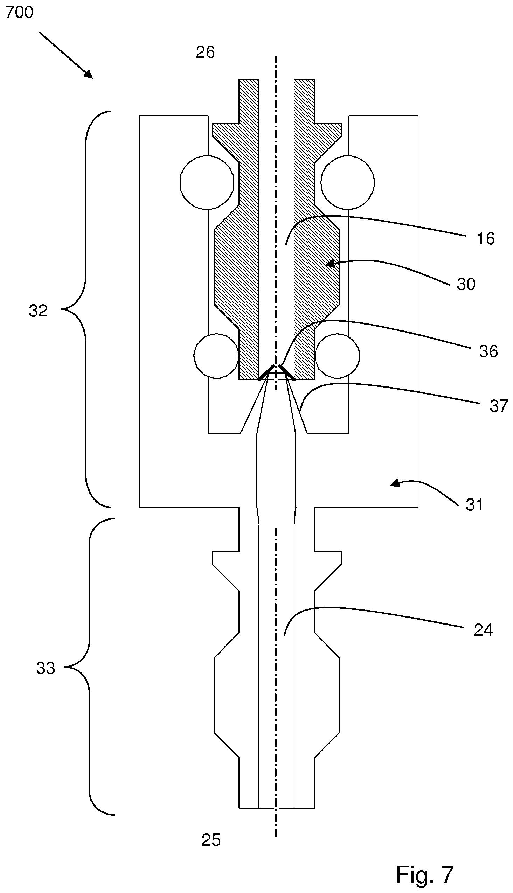

Die Figur 7 zeigt einen Konnektor 700, welcher dem Konnektor 600 der Figur 6 ähnelt, sich jedoch darin unterscheidet, dass anstelle durch den Port 34 der Strömungskanal 16 des Konnektorelements 30 durch ein Absperrorgan in Form einer hydrophoben Membran 36 verschlossen ist, welche auf der Flüssigkeitsleitungsseite 25 bzw. auf der dem Konnektorelement 31 zugewandten Seite des Konnektorelements 30 angeordnet oder ausgebildet ist. Die Membran 36 ist selbsttätig geschlossen und sperrt damit den Strömungskanal 16 bei Diskonnektion sowohl in Auströmrichtung als auch in Einströmrichtung. Sie gibt den Strömungskanal 16 in beide Richtungen frei, sobald eine Konnektion durch bestimmungsgemäßes Koppeln von Konnektorelement 30 und Konnektorelement 31 bewirkt ist. Dies wird durch ein Öffnungselement 37 in Form einer Anstechnadel, das am Konnektorelement 31 ausgebildet ist, bewirkt, indem dieses bei bestimmungsgemäßer Konnektion die Membran 36 durchsticht und öffnet. Beim Entkoppeln der Konnektorelemente 30 und 31 schließt die hydrophobe Membran 36 von selbst fluiddicht ab.

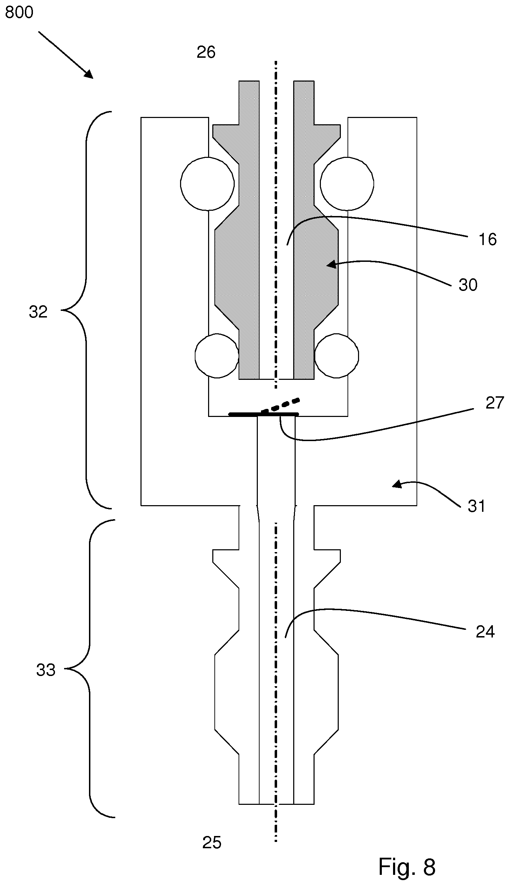

Die Figuren 8 bis 12 zeigen jeweils eine Konnektoreinheit 800, 900, 1000, 1100 bzw. 1200, die jeweils der Konnektoreinheit 600 der Figur 6 sowie der Konnektoreinheit 700 der Figur 7 im Wesentlichen entsprechen, so dass auf die dortigen Beschreibungen verwiesen wird.

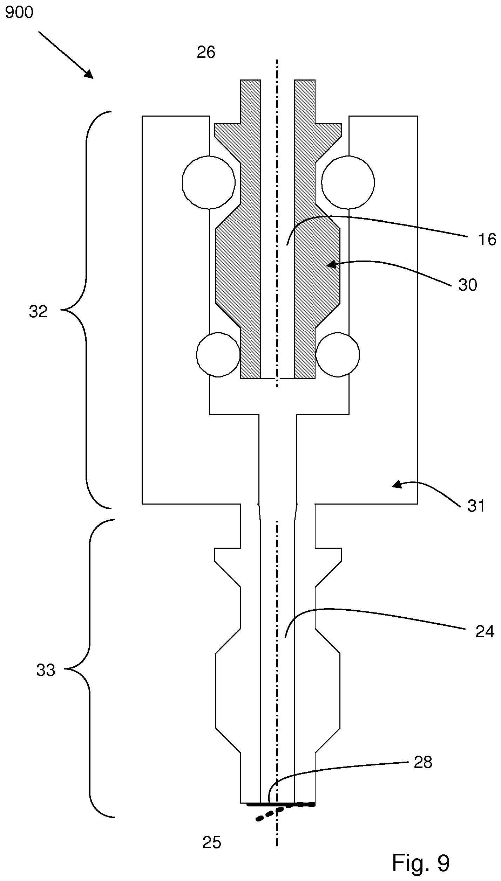

Anders als die vorgenannten Ausführungsformen der Figuren 6 und 7 ist an dem Konnektorelement 31 der Ausführungsformen der Figuren 8 und 9 ähnlich wie in der Ausführungsform der Figuren 3 und 4 ein Rückschlagventil 27 bzw. 28 angeordnet, das den Strömungskanal 24 sperrt bzw. frei gibt. Es wird auf die entsprechenden Ausführungen zu den Figuren 3 und 4 verwiesen. Bei der Ausführungsform der Figur 8 ist das Rückschlagventil 27 am weiblichen Kupplungsabschnitt 32 auf der dem Konnektorelement 30 zugewandten Seite des Konnektorelements 31 angeordnet und öffnet bei entsprechendem Druck nach Innen, d.h. zum Konnektorelement 30 hin. Bei der Ausführungsform der Figur 9 ist das Rückschlagventil 28 am männlichen Kupplungsabschnitt 33 auf der Flüssigkeitsleitungsseite 25 angeordnet und öffnet bei entsprechendem Druck nach Außen, d.h. zu einer der Leitungen 3, 9, 10, 12 hin.

Der wesentliche Vorteil der in den Figuren 8 und 9 gezeigten Ausführungsformen ist darin zu sehen, dass das Konnektorelement 31 gewissermaßen als eine Art Adapter fungiert und somit ermöglicht, dass weder der Anschluss 30 am Dialysator noch das Konnektorelement 23 an der anzuschließenden Leitung 3, 9, 10, 12 in irgendeiner Form verändert oder angepasst werden müssen. Somit kann mit dem Konnektor 800 bzw. 900 jedes derzeit verfügbare System nachgerüstet werden. Das Konnektorelement 31 wird einerseits am Dialysatoranschluss 30 angebracht und verschließt diesen. Beim Verbinden des Konnektorelements 31 mit einem Konnektorelement 23 der Leitung 3, 9, 10, 12 und anschließendem Pumpen von Flüssigkeit durch die Leitungen wird die Fluidverbindung zwischen Dialysator 5 und Leitung 3, 9, 10, 12 geöffnet bzw. hergestellt, da der Förderdruck das bzw. die Rückschlagventile 27, 28, 29 öffnet.

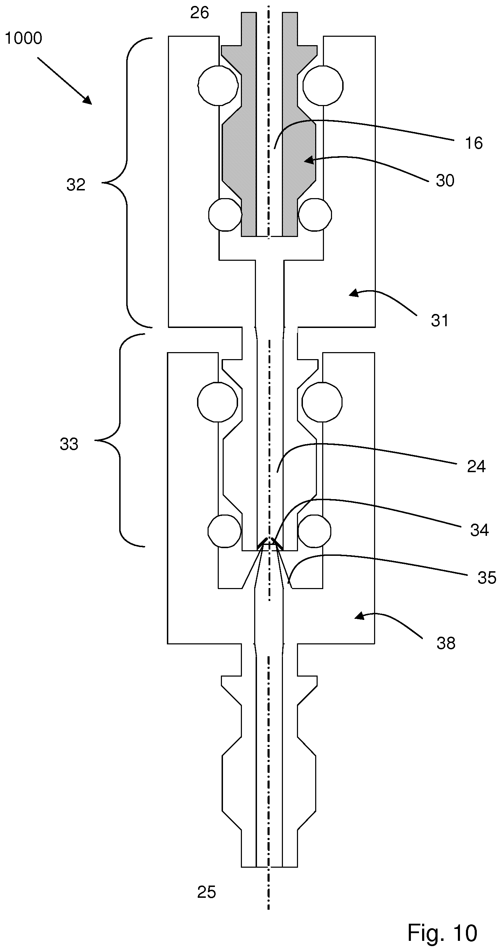

Bei der Ausführungsform der Figur 10 ist in dem Konnektorelement 31 der Konnektoreinheit 1000 im Bereich von dessen den Leitungen 3, 9, 10, 12 zugewandtem Ende ähnlich der Ausführungsform der Figur 6 ein Port 34 angeordnet. Es wird auf die diesbezüglichen Erläuterungen zur Ausführungsform der Figur 6 verwiesen. Leitungsseitig ist auf das Konnektorelement 31 ein weiteres Konnektorelement 38 angekoppelt, ebenfalls nach Art einer Hansenkonnektion, das wie auch das Konnektorelement 31 einen weiblichen Kupplungsabschnitt 32 und einem männlichen Kupplungsabschnitt 33 aufweist.

Bei der Ausführungsform der Figur 11 ist in dem Konnektorelement 31 der Konnektoreinheit 1100 im Bereich von dessen den Leitungen 3, 9, 10, 12 zugewandtem Ende 25 ähnlich der Ausführungsform der Figur 6 ein Ventilsystem 29 angeordnet, das druckabhängig und richtungsunabhängig arbeitet. Es wird auf die diesbezüglichen Erläuterungen zur Ausführungsform der Figur 6 verwiesen.

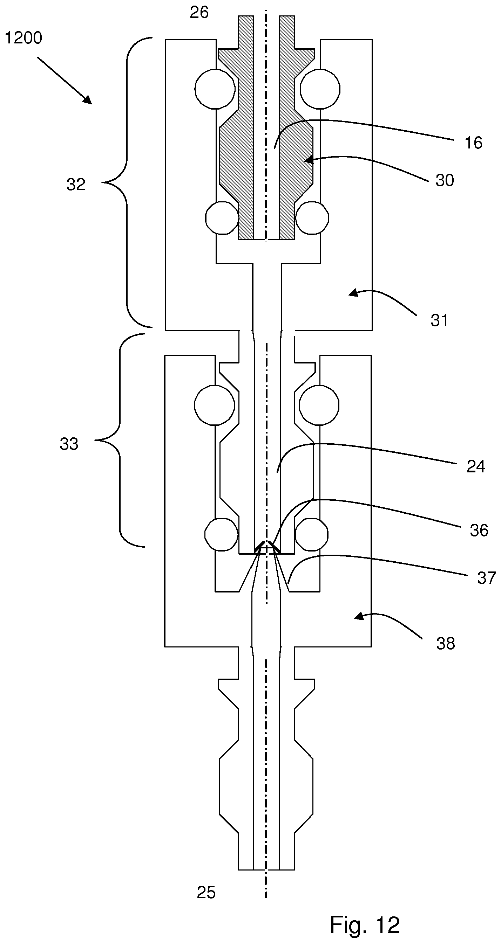

Bei der Ausführungsform der Figur 12 ist in dem Konnektorelement 31 der Konnektoreinheit 1200 im Bereich von dessen den Leitungen 3, 9, 10, 12 zugewandtem Ende 25 ähnlich der Ausführungsform der Figur 7 eine hydrophobe Membran 36 angeordnet. Es wird auf die diesbezüglichen Erläuterungen zur Ausführungsform der Figur 7 verwiesen. Leitungsseitig ist auf das Konnektorelement 31 ein weiteres Konnektorelement 38 angekoppelt, ebenfalls nach Art einer Hansenkonnektion, das wie auch das Konnektorelement 31 einen weiblichen Kupplungsabschnitt 32 und einem männlichen Kupplungsabschnitt 33 aufweist.

Der wesentliche Vorteil der in den Figuren 10 und 12 gezeigten Ausführungsformen ist darin zu sehen, dass die beiden Konnektorelemente 31 und 38 gewissermaßen als eine Art Adapter oder Adapterset fungieren und somit ermöglichen, dass weder der Anschluss 30 am Dialysator noch das Konnektorelement 23 an der anzuschließenden Leitung 3, 9, 10, 12 in irgendeiner Form verändert oder angepasst werden müssen. Somit kann mit dem Konnektor 1000 bzw. 1200 bzw. mit den Konnektorelementen 31 und 38 jedes derzeit verfügbare System nachgerüstet werden. Das Konnektorelement 31 wird am Dialysatoranschluss 30 angebracht und verschließt diesen und das Konnektorelement 38 wird an der Leitung 3, 9, 10, 12, genauer gesagt an dem Konnektorelement 23, angebracht. Beim Verbinden der beiden Konnektorelemente wird die Fluidverbindung zwischen Dialysator 5 und Leitung 3, 9, 10, 12 geöffnet bzw. hergestellt, da das Konnektorelement 38 beim Verbinden das Sperrorgan 34 bzw. 36 (zwanghaft) öffnet.

## Patentansprüche

1. Konnektor (500; 1100) zum Verbinden eines Dialysators (5) mit einer flüssigkeitsführenden Leitung (3, 9, 10, 12), wobei der Konnektor (500; 1100) einen Kupplungsabschnitt (15, 33), insbesondere einen männlichen Hansenkonnektorabschnitt, zur flüssigkeitsdichten Verbindung mit einem Konnektorelement (23), insbesondere einer weiblichen Hansenkonnektoraufnahme, der Leitung (3, 9, 10, 12) aufweist,
wobei
in einem Strömungskanal (16; 24) des Konnektors ein Absperrorgan (29) integriert ist, das unterhalb eines vorbestimmten Drucks den Strömungsquerschnitt des Strömungskanals (16, 24) absperrt,
**dadurch gekennzeichnet, dass**
das Absperrorgan (29) druckabhängig und strömungsrichtungsunabhängig durch eine anliegende Fluiddruckdifferenz zu öffnen ist.

2. Konnektor (500; 1100) nach Anspruch 1, **dadurch gekennzeichnet, dass** das Absperrorgan ein bidirektionales Rückschlagventil (29) aufweist.

3. Konnektor (1100) nach Anspruch 1 oder 2, **dadurch gekennzeichnet, dass** der Konnektor ferner einen Kupplungsabschnitt (32), insbesondere Schnellkupplungsabschnitt, zur lösbaren und flüssigkeitsdichten Verbindung mit dem Dialysator (5) aufweist.

4. Konnektor (1100) nach einem der vorhergehenden Ansprüche, **dadurch gekennzeichnet, dass** der Konnektor zwei miteinander kuppelbare sowie voneinander entkuppelbare Konnektorelemente (30, 31) aufweist, wobei insbesondere das erste Konnektorelement (30) zur gegenseitigen Kopplung einen männlichen Kupplungsabschnitt und das zweite Konnektorelement (31) zur gegenseitigen Kopplung einen weiblichen Kupplungsabschnitt (32) aufweist.

5. Konnektor nach einem der Ansprüche 1 bis 3, **dadurch gekennzeichnet, dass** der Konnektor zwei miteinander kuppelbare sowie voneinander entkuppelbare Konnektorelemente aufweist, wobei das erste Konnektorelement (31) eine weibliche Hansenkonnektoraufnahme (32) zur Kopplung mit einem entsprechenden an dem Anschluss (15) des Dialysators (5) ausgebildeten männlichen Hansenkonnektorelement (30) aufweist und das zweite Konnektorelement ein männliches Hansenkonnektorelement zur Kopplung mit einer entsprechenden an der anzuschließenden Leitung ausgebildeten weiblichen Hansenkonnektoraufnahme aufweist, wobei das Sperrorgan (29) in dem ersten Konnektorelement (31) vorgesehen ist.

6. Dialysator (5) für eine extrakorporale Blutbehandlung mit einem Blutzuleitungsanschluss (4), einem Blutableitungsanschluss (8), einem Dialysierflüssigkeitszuleitungsanschluss (11) und einem Dialysierflüssigkeitsableitungsanschluss (13), wobei ein Konnektor (500; 1100) nach einem der vorstehenden Ansprüche einstückig mit dem Dialysator ausgebildet ist oder an zumindest einem Anschluss (4, 8, 11, 13) des Dialysators (5) lösbar angebracht ist.

7. Verfahren zum Spülen eines Dialysators (5) für eine extrakorporale Blutbehandlung, welcher einen Blutzuleitungsanschluss (4), einen Blutableitungsanschluss (8), einen Dialysierflüssigkeitszuleitungsanschluss (11) und einen Dialysierflüssigkeitsableitungsanschluss (13) aufweist, wobei an zumindest einem Anschluss (4, 8, 11, 13) des Dialysators (5), vorzugsweise an jedem Anschluss (4, 8, 11, 13) des Dialysators (5), ein Konnektor (500; 1100) nach einem der vorstehenden Ansprüche, angeordnet ist und der Dialysator (5) gespült wird, indem eine Spülflüssigkeitszuleitung (10) mit dem an den Blutzuleitungsanschluss (4) angeordneten Konnektor (500; 1100) gekoppelt wird und eine Spülflüssigkeitsableitung (12) mit dem an den Blutableitungsanschluss (4) angeordneten Konnektor (500; 1100) gekoppelt wird.

## Claims

1. A connector (500; 1100) for connecting a dialyser (5) to a fluid-carrying line (3, 9, 10, 12), wherein the connector (500; 1100) includes a coupling portion (15, 33), especially a male Hansen connector portion, for fluid-tight connection to a connector element (23), especially a female Hansen connector adapter, of the line (3, 9, 10, 12),
wherein
in a flow channel (16; 24) of the connector, a shut-off device (29) is integrated which shuts off the flow cross-section of the flow channel (16, 24) below a predetermined pressure,
**characterized in that**
the shut-off device (29) is to be opened in a pressure-dependent manner and independently of the flow direction by an applied fluid pressure difference.

2. The connector (500; 1100) according to claim 1, **characterized in that** the shut-off device includes a bidirectional check valve (29).

3. The connector (1100) according to claim 1 or 2, **characterized in that** the connector further includes a coupling portion (32), especially a quick-release coupling portion, for releasable and fluid-tight connection to the dialyser (5).

4. The connector (1100) according to any one of the preceding claims, **characterized in that** the connector includes two connector elements (30, 31) adapted to be coupled to each other and to be uncoupled from each other, wherein especially the first connector element (30) includes a male coupling portion for mutual coupling and the second connector element (31) includes a female coupling portion (32) for mutual coupling.

5. The connector according to any one of the claims 1 to 3, **characterized in that** the connector includes two connector elements adapted to be coupled to each other and to be uncoupled from each other, wherein the first connector element (31) includes a female Hansen connector adapter (32) for coupling to a corresponding male Hansen connector element (30) formed at the port (15) of the dialyser (5) and the second connector element includes a male Hansen connector element for coupling to a corresponding female Hansen connector adapter formed at the line to be connected, the shut-off device (29) being provided in the first connector element (31).

6. A dialyser (5) for an extracorporeal blood treatment comprising a blood supply line port (4), a blood drain line port (8), a dialysis fluid supply line port (11) and a dialysis fluid drain line port (13), wherein a connector (500; 1100) according to any one of the preceding claims is formed integrally with the dialyser or is detachably arranged on at least one port (4, 8, 11, 13) of the dialyser (5).

7. A method of flushing a dialyser (5) for extracorporeal blood treatment, which comprises a blood supply line port (4), a blood drain line port (8), a dialysis fluid supply line port (11) and a dialysis fluid drain line port (13), wherein a connector (500; 1100) according to any one of the preceding claims is arranged on at least one port (4, 8, 11, 13) of the dialyser (5), preferably on each port (4, 8, 11, 13) of the dialyser (5), and the dialyser (5) is flushed by coupling a washing fluid supply line (10) to the connector (500; 1100) arranged at the blood supply line port (4) and by coupling a washing fluid drain line (12) to the connector (500; 1100) arranged at the blood drain line port (4).

## Revendications

1. Connecteur (500, 1100) pour connecter un dialyseur (5) à une conduite de transport de liquide (3, 9, 10, 12), dans lequel le connecteur (500, 1100) présente une section de couplage (15, 33), en particulier une section de connecteur Hansen mâle, destinée à être connectée de manière étanche avec un élément de connecteur (23), en particulier avec une entrée de connecteur Hansen femelle de la conduite (3, 9, 10, 12),
dans lequel
dans un canal d'écoulement (16 ; 24) du connecteur, un obturateur (29) est intégré, qui obstrue la section transversale d'écoulement du canal d'écoulement (16, 24) en dessous d'une pression prédéterminée,
**caractérisé en ce que**
l'obturateur (29) dépend de la pression et peut être ouvert indépendamment de la direction d'écoulement par une différence de pression de fluide appliquée.

2. Connecteur (500, 1100) selon la revendication 1, **caractérisé en ce que** l'obturateur présente un clapet anti-retour bidirectionnel (29).

3. Connecteur (1100) selon la revendication 1 ou 2, **caractérisé en ce que** le connecteur présente en outre une section de couplage (32), en particulier une section de couplage rapide, pour une connexion de manière amovible et étanche avec le dialyseur (5).

4. Connecteur (1100) selon l'une quelconque des revendications précédentes, **caractérisé en ce que** le connecteur présente deux éléments de connecteur (30, 31) pouvant être couplés l'un à l'autre et désaccouplés l'un de l'autre, dans lequel en particulier le premier élément de connecteur (30) présente une section de couplage mâle pour un couplage mutuel, et le second élément de connecteur (31) présente une section de couplage femelle (32) pour un couplage mutuel.

5. Connecteur selon l'une des revendications 1 à 3, **caractérisé en ce que** le connecteur présente deux éléments de connecteur pouvant être couplés l'un à l'autre et désaccouplés l'un de l'autre, dans lequel le premier élément de connecteur (31) présente une entrée de connecteur Hansen femelle (32) destinée à un couplage à un élément de connecteur Hansen mâle (30) correspondant formé au niveau du raccord (15) du dialyseur (5), et le second élément de connecteur présente un élément de connecteur Hansen mâle (30) destiné à un couplage à une entrée de connecteur femelle correspondante formée au niveau de la conduite à connecter, dans lequel l'obturateur (29) est prévu dans le premier élément de connecteur (31).

6. Dialyseur (5) pour un traitement de sang extracorporel avec un raccord d'alimentation de sang (4), un raccord d'évacuation de sang (8), un raccord d'alimentation en liquide de dialyse (11) et un raccord d'évacuation de fluide de dialyse (13), dans lequel un connecteur (500 ; 1100) selon l'une quelconque des revendications précédentes est formé d'un seul tenant avec le dialyseur ou est fixé de manière amovible à au moins un raccord (4, 8, 11, 13) du dialyseur (5).

7. Procédé de lavage d'un dialyseur (5) pour un traitement de sang extracorporel, qui présente un raccord d'alimentation de sang (4), un raccord d'évacuation de sang (8), un raccord d'alimentation en liquide de dialyse (11) et un raccord d'évacuation de liquide de dialyse (13), dans lequel sur au moins un raccord (4, 8, 11, 13) du dialyseur (5), de préférence sur chaque raccord (4, 8, 11, 13) du dialyseur (5), est agencé un connecteur (500, 1100) selon l'une quelconque des revendications précédentes, et le dialyseur (5) est lavé en couplant une conduite d'alimentation en liquide de lavage (10) au connecteur (500 ; 1100) agencé sur le raccord d'alimentation de sang (4) et en couplant une conduite d'évacuation de liquide de lavage (12) au connecteur (500 ; 1100) agencé sur le raccord d'évacuation de sang (4).
